# FASCICULE DE BREVET EUROPEEN

(11) **EP 0 819 427 B1**
(45) Date de publication et mention de la délivrance du brevet: **06.05.2004**
(21) Numéro de dépôt: 97401383.1
(22) Date de dépôt: 17.06.1997
(51) Int. Cl.: A61K 7/48

(54) **Utilisation de l'iodo-3 propynyl-2 butylcarbamate dans une composition cosmétique et/ou dermatologique comme actif pour le traitement de la séborrhée**
Verwendung von 2(3-Iod-2-Propinyl)-Butylcarbamat in einer kosmetischen und/oder dermatologischen Zusammensetzung zur Behandlung von Seborrhea
Use of 2(3-iodo-2-propynyl)-butylcarbamate in a cosmetic and/or dermatological composition as active ingredient for the treatment of seborrhea

(30) Priorité: 16.07.1996 FR 9608887
(43) Date de publication de la demande: 21.01.1998
(73) Titulaire: L'OREAL, 75008 Paris (FR)
(72) Inventeur: Renault, Béatrice, 94410 Saint Maurice (FR); Ayache, Liliane, 75012 Paris (FR); Cupferman, Sylvie, 75015 Paris (FR)
(74) Mandataire: Lhoste, Catherine

(56) Documents cités:
- EP-A- 0 705 595
- WO-A-92/09263
- WO-A-93/04669
- WO-A-95/29588
- WO-A-96/24329

## Description

La présente invention a pour objet l'utilisation de l'iodo-3 propynyl-2 butylcarbamate dans une composition cosmétique ou dermatologique, comme actif pour traiter la séborrhée et les dermatoses qui lui sont associées, notamment la dermite séborrhéique, l'acné et les pellicules.

La secrétion de sébum est un phénomène normal et utile à la peau comme à la chevelure. Cependant, l'hypersecrétion de sébum, la séborrhée, entraîne des désagréments et parfois une pathologie cutanée, notamment une peau grasse et même acnéique, et un état pelliculaire du cuir chevelu. L'hypersécrétion sébacée et la perturbation de la kératinisation du follicule pilosébacé ont pour conséquences l'obstruction du follicule pilosébacé et la formation de lésions rétentionnelles ou comédons.

Ces pathologies relèvent notamment de la colonisation de la peau ou du follicule pileux par la levure *Pityrosporum ovale.* Dans le cas particulier de l'acné, cette levure est le plus souvent associée à des germes du genre *Propionibacterium* tels que *Propionibacterium acnes, Propionibacterium granulosum, Propionibacterium avidum*.

Pour lutter contre ces agents pathogènes, on utilise couramment des actifs tels que l'omadine de sodium ou de zinc, l'octopirox, voire les goudrons tels que l'ichthyol, le coal tar et tout goudron d'origine végétal ou minéral. Mais l'utilisation de ces actifs n'est pas dénuée d'effets secondaires. Ainsi, l'omadine de sodium présente une toxicité non négligeable, notamment par voie topique. On lui préfère de ce fait l'omadine de zinc. Toutefois, ce dernier présente l'inconvénient d'être insoluble, ce qui rend son utilisation difficile. Par ailleurs, l'octopirox s'est révélé insuffisamment efficace, notamment dans certains véhicules où son activité est inhibée. Quant aux goudrons utilisés dans la passé, ils ne permettent pas d'obtenir des résultats probants et peuvent, en outre, être toxiques. De plus, leur odeur rend leur utilisation rédhibitoire.

On constate donc que subsiste le besoin d'actifs topiques ayant un effet sur les pathologies liées à *Pityrosporum ovale,* ayant une action au moins aussi efficace que les composés de l'art antérieur, notamment sur l'hypersécrétion sébacée et par voie de conséquence sur les dermatoses qui lui sont associées, tout en ne présentant pas les inconvénients des composés connus.

C'est ce qu'apporte la présente invention.

La demanderesse a découvert que l'iodo-3 propynyl-2 butylcarbamate présentait une forte activité sur *Pityrosporum ovale* et pouvait donc être utilisé dans une composition cosmétique ou dermatologique en tant qu'actif anti-séborrhéique.

L'iodo-3 propynyl-2 butylcarbamate est connu comme fongicide, notamment pour le bois (voir article de Lee et al., Bokin Bobai, 1990, 18 (8), p. 365-370). Par ailleurs, il est décrit comme étant un conservateur approprié pour les produits cosmétiques par la société Lonza qui le commercialise sous la dénomination « Glycacil ». Toutefois, son utilisation pour agir sur les pathologies liées à *Pityrosporum ovale* et notamment pour traiter la séborrhée n'a jamais été décrite.

La présente invention a donc pour objet l'utilisation de l'iodo-3 propynyl-2 butylcarbamate pour la fabrication d'un médicament destiné à traiter la dermite séborrhéique.

Aussi, la présente invention a encore pour objet un procédé de traitement cosmétique des pellicules, consistant à appliquer sur les cheveux et/ou le cuir chevelu, une composition contenant de l'iodo-3 propynyl-2 butylcarbonate.

De préférence, l'iodo-3 propynyl-2 butylcarbamate est utilisé en mélange avec un composé tel qu'un solvant, un excipient ou un actif complétant son activité anti-séborrhéique.

Aussi, l'invention a encore pour objet une composition cosmétique et/ou dermologique anti-séborrhéique, caractérisée en ce qu'elle contient, dans un milieu cosmétiquement et/ou dermatologiquement acceptable, une quantité efficace d'iodo-3 propynyl-2 butylcarbamate et l'octopinox.

Ainsi, dans la composition selon l'invention, l'iodo-3 propynyl-2 butylcarbamate peut être utilisé sous forme d'un mélange avec un ou plusieurs solvants choisis parmi les esters d'acide gras et de polyéthylène glycol et les polyéthylènes glycols de faible poids moléculaire, c'est-à-dire de poids moléculaire inférieur ou égal à 450. On entend par acide gras les acides comportant de 12 à 22 atomes de carbone. L'ester peut dériver d'un acide gras ou d'un mélange d'acides gras.

Comme esters d'acide gras et de polyéthylène glycol, on peut citer par exemple les mono- et di-cocoates de polyéthylène glycol. Comme polyéthylène glycols de poids moléculaire inférieur ou égal à 450, on peut citer par exemple le polyéthylène glycol (4 OE) ou PEG-4, le polyéthylène glycol (6 OE) ou PEG-6, le polyéthylène glycol (8 OE) ou PEG-8 et le polyéthylène glycol (9 OE) ou PEG-9.

Selon une réalisation particulière de l'invention, l'iodo-3 propynyl-2 butylcarbamate est utilisé en mélange avec le monococoate de polyéthylène glycol, le dicocoate de polyéthylène glycol et le polyéthylène glycol (4 OE) ; il s'agit plus particulièrement du mélange vendu par la société Lonza sous la dénomination « Glycacil L » où les constituants se trouvent dans un rapport 10/40/40/10 : 10 % d'iodo-3 propynyl-2 butylcarbamate, 40 % de monococoate de polyéthylène glycol, 40 % de dicocoate de polyéthylène glycol et 10 % de polyéthylène glycol (4 OE).

L'iodo-3 propynyl-2 butylcarbamate peut être utilisé également dans la composition selon l'invention sous forme solide, notamment en mélange avec un carbonate ou bicarbonate de métal alcalin ou de métal alcalino-terreux, et plus particulièrement du bicarbonate de sodium.

La quantité de solvant ou d'excipient peut aller par exemple de 0,01 à 20 % et de préférence de 0,1 à 10 % du poids total de la composition.

Par ailleurs, la composition selon l'invention peut contenir l'iodo-3 propynyl-2 butylcarbamate en mélange avec un ou plusieurs actifs susceptibles de compléter l'effet de l'iodo-3 propynyl-2 butylcarbamate dans le traitement de la séborrhée et des dermatoses associées, et notamment de l'acné. Comme actifs de ce type, on peut citer notamment les agents anti-inflammatoires tels que le peroxyde de benzoyle, les antibiotiques tels que la clindamycine et l'érythromycine, les agents antiseptiques tels que l'octopirox et les actifs kératolytiques tels que les α-hydroxy-acides ou les β-hydroxy-acides, l'acide rétinoïque et ses dérivés, le rétinol et ses dérivés tels que le palmitate, l'acétate ou le propionate de rétinol. Selon un mode préféré de réalisation de l'invention, cet actif est un actif kératolytique, et notamment un α-hydroxy-acide ou un β-hydroxy-acide.

Comme α-hydroxy-acides, on peut citer en particulier les acides glycolique, lactique, malique, tartrique, citrique, mandélique. Comme β-hydroxy-acides, on peut citer l'acide salicylique ainsi que ses dérivés comme l'acide n-octanoyl-5-salicylique et l'acide n-dodécanoyl-5-salicylique, les acides hydroxy-2 alcanoïques et leurs dérivés comme l'acide hydroxy-2-méthyl-3-benzoïque et l'acide hydroxy-2-méthoxy-3-benzoïque.

La quantité d'actif(s) anti-inflammatoire(s) et/ou antibiotique(s) et/ou d'agent(s) antiseptique(s) et/ou d'actif(s) kératolytique(s) peut aller par exemple de 0,01 à 20 % et de préférence de 0,1 à 5 % du poids total de la composition.

L'iodo-3 propynyl-2 butylcarbamate est utilisé, selon la présente invention, en une quantité allant de préférence de 0,005 à 5 % et mieux de 0,01 à 0,5 % du poids total de la composition.

Les compositions selon l'invention contiennent un milieu cosmétiquement et/ou dermatologiquement acceptable, c'est-à-dire compatible avec la peau, les muqueuses, les cheveux et le cuir chevelu. Elles peuvent se présenter sous toutes les formes galéniques appropriées pour une application topique, notamment sous forme de solutions aqueuses, hydroalcooliques ou huileuses, de gels aqueux, hydroalcooliques ou huileux, de produits anhydres pâteux ou solides, d'émulsions obtenues par dispersion d'une phase grasse dans une phase aqueuse (H/E) ou inversement (E/H), de suspensions, de microémulsions, de microcapsules, de microparticules, ou encore de dispersions vésiculaires de type ionique (liposomes) et/ou non ionique. Ces compositions sont préparées selon les méthodes usuelles dans les domaines considérés.

Les compositions selon l'invention peuvent être également utilisées sous forme de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des produits pour la peau tels que des gels ou crèmes de nettoyage ou de traitement, des lotions ou laits purifiants, des sticks camouflants, des lotions pour les cheveux ou des shampooings antipelliculaires, des compositions anti-chute pour le cuir chevelu.

Les compositions de l'invention peuvent comprendre les adjuvants classiquement mis en oeuvre dans les domaines considérés comme les corps gras, les solvants organiques, les agents solubilisants, les agents épaississants et gélifiants, les adoucissants, les antioxydants, les opacifiants, les agents stabilisants, les agents moussants, les parfums, les émulsionnants ioniques ou non, les charges, les séquestrants et les chélateurs, les conservateurs, les parfums, les filtres, les huiles essentielles, les matières colorantes, les pigments, les actifs hydrophiles ou lipophiles, les vésicules lipidiques ou tout autre ingrédient habituellement utilisé en cosmétique.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et éventuellement le coémulsionnant sont présents, dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme huiles utilisables dans l'invention, on peut citer les huiles minérales (isoparaffine), les huiles végétales (huile d'amandes d'abricot), les huiles animales, les huiles de synthèse (huile de purcellin, polyisobutène hydrogénée), les huiles siliconées (cyclopentadiméthylsiloxane) et les huiles fluorées. On peut aussi utiliser comme matières grasses des alcools gras, des acides gras (acide stéarique), des cires (cire de carnauba, ozokérite).

Comme émulsionnants utilisables dans l'invention, on peut citer par exemple le monostéarate d'éthylène glycol, le tristéarate de sorbitane, le mélange palmito-stéarate de glycol / stéarate de polyéthylène glycol (6 OE) / stéarate de polyéthylène glycol (32 OE) vendu sous la dénomination « Tefose 63 » par la société Gattefosse, et la lécithine hydrogénée.

Comme gélifiants ou épaississants, on peut citer les gommes naturelles (gomme de xanthane), les polysaccharides (hydroxypropylmethylcellulose, carboxyméthylcellulose), les polymères carboxyvinyliques (carbomer), les copolymères acryliques, les polyacrylamides et notamment le mélange de polyacrylamide, C₁₃-C₁₄-Isoparaffine et Laureth-7, vendu sous la dénomination de Sepigel 305 par la société Seppic, les dérivés de sucres oxyéthylénés tels que le méthylglucose oxyéthyléné.

Comme agents moussants, on peut citer par exemple le N-carboxyéthoxyéthyl N-cocoylamidoéthyl aminoacétate N-di-sodique, le lauryl éther sulfate de sodium, le lauroyl sarcosinate de sodium, le triéthanolamine lauryl sulfate et encore le mélange de cocoyl isethionate de sodium et d'acides gras de coprah.

Comme charges, on peut citer notamment les copolymères acryliques tels que le copolymère diméthacrylate d'éthylène glycol/ methacrylate de lauryle vendu par la société Dow Corning sous la dénomination de Polytrap.

Par ailleurs, comme actifs hydrophiles, on peut utiliser par exemple les protéines ou les hydrolysats de protéine, les acides aminés, les polyols (glycérine, propylène glycol), l'urée, l'allantoïne, les sucres et les dérivés de sucre, les vitamines, l'amidon, les extraits bactériens ou végétaux.

Quand les compositions selon l'invention sont plus particulièrement destinées à être utilisées lors d'exposition au soleil, elles contiennent, en outre, de manière appropriée au moins un filtre, de façon à préserver l'efficacité de l'actif selon l'invention, en protégeant la peau des effets néfastes des rayons solaires. Comme filtres, on peut citer les filtres organiques ou les pigments qui peuvent être sous forme de nanoparticules (nanopigments) ou non. Des exemples de filtres organiques sont les dérivés sulfonés ou sulfonatés de la benzophénone, les dérivés sulfoniques ou sulfonatés du benzylidène camphre et les acrylates tels que l'octocrylène. La quantité de filtre dépend de la protection solaire souhaitée. Elle peut aller par exemple de 0,01 à 10 % et de préférence de 0,1 à 5 % du poids total de la composition.

Les exemples suivants illustrent l'invention. Les pourcentages sont donnés en poids.

### Exemple 1 : Gel moussant pour peaux grasses séborrhéiques

- copolymère d'alcool de suif hydrogéné oxyéthyléné (60 OE) / myristyl glycol (solubilisant) (Elfacos GT 282 S vendu par la société Akzo) 0,9 %
- iodo-3 propynyl-2 butylcarbamate 0,1 %
- glycérine 3 %
- N-carboxyéthoxyéthyl N-cocoylamidoéthyl aminoacétate N-di-sodique à 38 % dans l'eau 5 %
- lauryl éther sulfate de sodium 14,3 %
- diéthanolamide d'acides gras de coprah (adoucissant) 0,7 %
- eau déminéralisée qsp 100 %

### Mode opératoire :

On disperse dans de l'eau déminéralisée, à 80°C, l'Elfacos GT 282S, puis on refroidit à 50°C. Par ailleurs, on disperse à 50°C dans de l'eau déminéralisée, les agents moussants, l'adoucissant et la glycérine sous agitation lente, puis on verse doucement cette préparation sous agitation lente dans la dispersion d'Elfacos GT 282S. On y ajoute ensuite à 40°C l'iodo-3 propynyl-2 butylcarbamate.

Le gel obtenu convient pour le traitement de la dermite séborrhéique, en application deux fois par jour sur le visage.

### Exemple 2 : Crème nettoyante moussante pour peaux acnéiques

- monostéarate d'éthylène glycol 2 %
- iodo-3 propynyl-2 butylcarbamate 0,1 %
- silicate de magnésium et d'aluminium hydraté (stabilisant) 1,7 %
- hydroxypropylmethylcellulose 0,8 %
- mélange de cocoyl isethionate de sodium et d'acides gras de coprah (Elfan AT 84 G vendu par la société Akzo) 15 %
- acide stéarique 1,25 %
- lauroyl sarcosinate de sodium à 30 % dans l'eau 10 %
- parfum 0,3 %
- eau déminéralisée qsp 100 %

### Mode opératoire :

On disperse l'Elfan AT 84 G dans de l'eau déminéralisée à 80°C. Après dissolution complète, on ajoute le sel de silicate de magnésium et d'aluminium hydraté. On refroidit à 60°C, puis on ajoute le lauroyl sarcosinate de sodium et le gel d'hydroxypropylmethylcellulose. On ajoute ensuite l'acide stéarique et le monostéarate d'éthylène glycol préalablement réchauffés au bain-marie. Enfin, on ajoute, à 40°C, l'iodo-3 propynyl-2 butylcarbamate et le parfum.

La crème obtenue convient pour le traitement de la peau acnéique, par application sur le visage deux fois par jour.

### Exemple 3 : Crème traitante pour l'acné et la dermite séborrhéique

- tristéarate de sorbitane 1 %
- iodo-3 propynyl-2 butyl carbamate 0,1 %
- carbomer 0,4 %
- gomme de xanthane 0,5 %
- Polytrap (Dow Corning) 1 %
- cyclopentadiméthylsiloxane (huile volatile) 6 %
- glycérine 3 %
- Tefose 63 (Gattefosse) (émulsionnant) 4 %
- eau déminéralisée qsp 100 %

### Mode opératoire :

On prépare le mélange A contenant le tristéarate de sorbitane, l'émulsionnant et le cyclopentadiméthylsiloxane à 60°C. Par ailleurs, on prépare le mélange B en faisant gonfler, dans l'eau déminéralisée à 70°C, le mélange de glycérol et de carbomer, puis la gomme de xanthane. On réalise l'émulsion à 65° C en versant le mélange A dans le mélange B sous agitation. On laisse refroidir à 40°C et on ajoute le Polytrap, puis l'iodo-3 propynyl-2- butyl carbamate.

La crème obtenue convient pour le traitement de la peau, par application sur le visage et le dos du corps une fois par jour.

### Exemple 4 : Crème traitante pour l'acné

On peut réaliser une crème identique à celle de l'exemple 3, en ajoutant, en outre, 7 % d'octocrylène (Uvinul N-5439-SG de BASF). On obtient une crème apte à traiter l'acné, même lors d'exposition au soleil.

### Exemple 5 : Gel traitant pour peaux séborrhéiques

- iodo-3 propynyl-2 butyl carbamate / mono- et di-cocoate de polyéthylène glycol / polyéthylène glycol (4 OE) (10/40/40/10) 0,25 %
- gomme de xanthane 1 %
- glycérine 2 %
- acide glycolique 2 %
- parfum 0,2 %
- alcool éthylique (solvant) 20 %
- mélange alcool butylique oxyéthyléné (26 OE) - oxypropyléné (26 OP) / huile de ricin hydrogénée oxyéthylénée (40 OE) dans l'eau (Solubilisant LRI vendu par la société Wackherr) (co-solvant) 1 %
- eau déminéralisée qsp 100 %

### Mode opératoire :

On fait gonfler la gomme de xanthane dans l'eau déminéralisée avec la glycérine à 70°C. Après refroidissement à 30°C, on ajoute l'acide glycolique, l'alcool éthylique, le co-solvant avec l'iodo-3 propynyl-2 butyl carbamate et le parfum.

Le gel obtenu convient pour le traitement de la peau séborrhéique, en application une à deux fois par jour sur le visage.

### Exemple 6 : Crème teintée traitante pour peaux acnéiques, contenant des liposomes

- stérols de soja oxyéthylénés (5 OE) 1,6 %
- lécithine hydrogénée 2,4 %
- iodo-3 propynyl-2 butyl carbamate / mono- et di-cocoate de polyéthylène glycol et polyéthylène glycol (4 OE) (10/40/40/10) 0,25 %
- huile d'amandes d'abricot 6 %
- oxyde de fer jaune (pigments) 0,45 %
- oxydes de fer brun (pigments) 0,4 %
- oxyde de fer noir (pigments) 0,07 %
- oxyde de titane (pigments) 5 %
- Polytrap (Dow Corning) 1 %
- polyacrylamide / C₁₃-C₁₄-Isoparaffine / Laureth-7 (Sepigel 305) 4 %
- cyclopentadiméthylsiloxane (huile volatile) 6 %
- glycérine 6 %
- propylène glycol 6 %
- eau déminéralisée qsp 100 %

### Mode opératoire :

Pour préparer les liposomes, on hydrate le mélange de stérols de soja oxyéthylénés (5 OE) et de lécithine hydrogénée dans de l'eau déminéralisée à 80°C pendant 2 heures et on passe deux fois cette préparation à l'homogénéisateur haute pression. Après avoir refroidi à 35°C la préparation de liposomes, on y ajoute l'huile d'amandes d'abricot et le cyclopentadiméthylsiloxane. On passe le mélange à l'homogénéisateur haute pression et on refroidit à 30°C. Par ailleurs, on prépare une dispersion des charges dans le propylène glycol et le glycérol. On disperse cette dispersion dans le mélange obtenu précédemment. On ajoute ensuite le Sepigel 305 et l'iodo-3 propynyl-2 butyl carbamate.

La crème obtenue est de couleur beige et elle convient pour le traitement de la peau, par application sur le visage deux fois par jour.

### Exemple 7 : Stick camouflant pour peaux grasses

- iodo-3 propynyl-2 butyl carbamate / mono-di-cocoate de polyéthylène glycol et polyéthylène glycol (4 OE) (10/40/40/10) 0,25 %
- cire de carnauba 8 %
- ozokerite 6 %
- oxyde de fer jaune (pigments) 2,5 %
- oxydes de fer brun (pigments) 1 %
- oxyde de fer noir (pigments) 0,5 %
- oxyde de titane (pigments) 20 %
- polydiméthylsiloxane / silice hydratée (agent de démoulage) 0,1 %
- isoparaffine qsp 100 %

### Mode opératoire :

On disperse dans le mélange de cires et d'huile, à 100°C, les oxydes et l'agent de démoulage, pendant 2 heures. On y ajoute l'iodo-3 propynyl-2 butyl carbamate juste avant le coulage.

Le stick obtenu peut être appliqué sur le visage plusieurs fois par jour.

### Exemple 8 : Lotion purifiante pour peaux acnéiques et pour cheveux pelliculeux

- iodo-3 propynyl-2 butyl carbamate 0,2 %
- glycérine 2 %
- alcool éthylique (solvant) 20 %
- alcool butylique oxyéthyléné (26 OE) oxypropyléné (26 OP) / huile de ricin hydrogénée oxyéthylénée (40 OE) dans l'eau(Solubilisant LRI vendu par la société Wackherr) (co-solvant) 1 %
- eau déminéralisée qsp 100 %

### Mode opératoire :

On mélange dans l'eau déminéralisée, la glycérine, l'alcool éthylique, le co-solvant et l'iodo-3 propynyl-2 butyl carbamate.

La lotion obtenue convient pour le nettoyage de la peau, par application sur le visage deux fois par jour.

### Exemple 9 : Shampooing antipelliculaire

- iodo-3 propynyl-2 butyi carbamate / mono- et di-cocoate de polyéthylène glycol et polyéthylène glycol (4 OE) (10/40/40/10) 0,25 %
- triéthanolamine lauryl sulfate à 40 % dans l'eau 37 %
- diéthanolamide d'acides gras de coprah (agent conditionneur de cheveux) 2 %
- carboxyméthylcellulose 0,5 %
- méthylglucose oxyéthyléné 2,5 %
- eau déminéralisée qsp 100 %

### Mode opératoire :

On disperse dans l'eau déminéralisée la carboxyméthylcellulose, et on ajoute l'agent conditionneur, le triéthanolamine lauryl sulfate et le méthylglucose oxyéthyléné. Après avoir refroidi à 30°C, on ajoute l'iodo-3 propynyl-2 butyl carbamate.

Le shampooing obtenu convient pour le lavage des cheveux pelliculeux et permet une nette amélioration de l'état du cuir chevelu.

### Exemple 10 : Poudre compacte pour le visage

- oxyde de fer jaune 0,2 %
- oxyde de fer brun, jaune 0,3 %
- oxyde de fer noir 0,06 %
- parfum 0,8 %
- carbonate de magnésium hydraté 20 %
- talc 73 %
- gomme arabique 0,5 %
- conservateur 0,006 %
- iodo-3 propynyl-2 butylcarbamate / bicarbonate de sodium (10/90) 0,2 %
- propylène glycol 4 %
- eau déminéralisée qsp 100 %

### Test :

Le test décrit ci-dessous met en évidence l'activité de l'iodo-3 propynyl-2 butylcarbamate sur *Pityrosporum ovale,* comparativement à l'octopirox, actif de l'état de la technique.

Les étapes pour réaliser ce test sont les suivantes :
1) Culture du microorganisme : *Pityrosporum ovale* est cultivé sur gélose en pente Sabouraud en présence de 10 % d'huile d'olive.
2) Préparation de l'inoculum : 48 heures avant le début du test, on effectue un repiquage de la souche et on laisse incuber pendant 48 heures à 35°C. La suspension obtenue titre à 10⁷ germes/ml.
3) Préparation de l'échantillon : On dépose dans un flacon de verre appelé pilulier, un bouillon Sabouraud et 2,5 % d'huile d'olive et on laisse incuber à 35°C pendant 24 heures. Puis, on ajoute 4 g de la composition à tester et on homogénéise. Parallèlement, on prépare un témoin pour vérifier que les germes sont dans des conditions de croissance favorables pendant la durée du test.
4) Inoculation : on introduit 4 ml d'inoculum dans le pilulier et on place le pilulier dans l'incubateur.
5) Prélèvements et dénombrements : après chaque temps de contact (2, 4, 6 et 24 heures), on homogénéise le contenu du pilulier, et on en prélève 1 ml. Après avoir déterminé la dilution appropriée pour pouvoir effectuer le comptage, on étale cette dilution en surface de boîtes de Pétri gélosées (gélose Sabouraud + 2,5% d'huile d'olive), puis on laisse incuber les boîtes de Pétri pendant 3 jours à l'étuve à 35°C et on effectue le comptage des colonies sur les boîtes contenant plus de 20 et moins de 200 colonies.

Les compositions testées sont les suivantes :

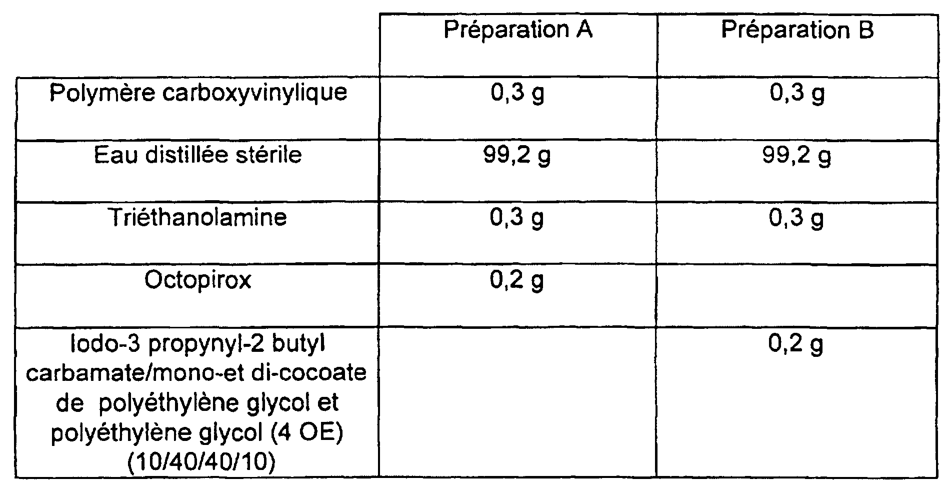

Les résultats obtenus sont indiqués dans le tableau suivant :

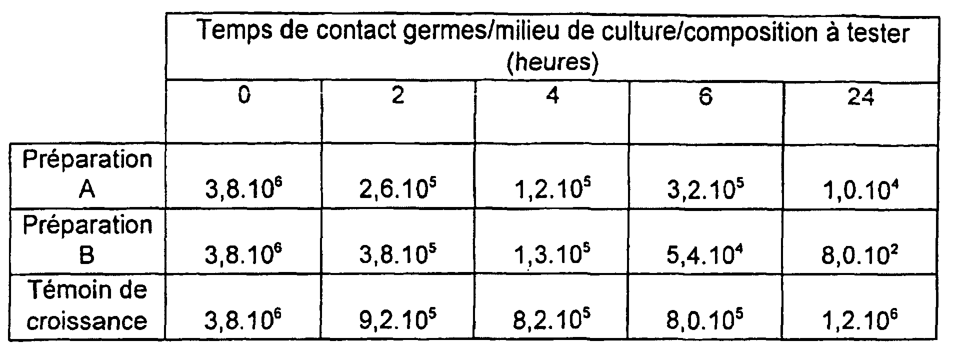

On constate dès 6 heures de contact, une décontamination supérieure avec la préparation B selon l'invention. Ce résultat est confirmé à 24 heures de contact puisque la préparation B a réduit la population initiale de près de 4 log₁₀, alors que la préparation A ne l'a réduit que de 2 log₁₀.

En outre, dans la préparation B, l'iodo-3 propynyl-2 butyl carbamate est à 10 % dans un mélange. Comme ce mélange est à 0,2 % dans la préparation B, la quantité de produit actif dans la préparation B est de 0,02 %. Dans la préparation A, le produit actif est pur et sa quantité est donc de 0,2 %. Compte-tenu de ces proportions, l'iodo-3 propynyl-2 butyl carbamate se révèle 1000 fois plus actif que l'octopirox.

## Revendications

1. Utilisation de l'iodo-3 propynyl-2 butylcarbamate pour la fabrication d'un médicament destiné à traiter la dermite séborrhéique.

2. Utilisation selon la revendication 1, **caractérisée en ce que** l'iodo-3 propynyl-2 butylcarbamate est présent en une quantité allant de 0,005 à 5 % en poids de la composition.

3. Utilisation selon la revendication 1 ou 2, **caractérisée en ce que** l'iodo-3 propynyl-2 butylcarbamate est présent en une quantité allant de 0,01 à 0,5 % en poids de la composition.

4. Utilisation selon l'une quelconque des revendications précédentes, **caractérisée en ce que** l'iodo-3 propynyl-2 butylcarbamate est en mélange avec un ou plusieurs solvants et/ou excipients.

5. Utilisation selon la revendication précédente, **caractérisée en ce que** le solvant est choisi parmi les esters d'acide gras et de polyéthylène glycol et les polyéthylène glycols de poids moléculaire inférieur ou égal à 450.

6. Utilisation selon l'une quelconque des revendications précédentes, **caractérisée en ce que** la composition comprend au moins un actif choisi les agents anti-inflammatoires, les antibiotiques, les agents antiseptiques, les actifs kératolytiques.

7. Utilisation selon la revendication précédente, **caractérisée en ce que** l'actif est choisi parmi les α-hydroxy-acides et les β-hydroxy-acides.

8. Utilisation selon la revendication 6, **caractérisée en ce que** l'agent antiseptique est l'octopirox.

9. Procédé de traitement cosmétique des pellicules, consistant à appliquer sur les cheveux et/ou le cuir chevelu, une composition contenant de l'iodo-3 propynyl-2 butylcarbamate.

10. Composition cosmétique et/ou dermatologique anti-séborrhéique, **caractérisée en ce qu'**elle contient, dans un milieu cosmétiquement et/ou dermatologiquement acceptable, une quantité efficace d'iodo-3 propynyl-2 butyl carbamate et l'octopirox.

11. Composition selon la revendication 10, **caractérisée en ce que** l'iodo-3 propynyl-2 butylcarbamate est présent en une quantité allant de 0,005 à 5 % du poids total de la composition.

## Patentansprüche

1. Verwendung von 3-Iod-2-propinylbutylcarbamat zur Herstellung eines Arzneimittels, das für die Behandlung von seborrhoischer Dermatitis vorgesehen ist.

2. Verwendung nach Anspruch 1, **dadurch gekennzeichnet, dass** das 3-Iod-2-propinylbutylcarbamat in einem Mengenanteil von 0,005 bis 5 Gew.-% der Zusammensetzung vorliegt.

3. Verwendung nach Anspruch 1 oder 2, **dadurch gekennzeichnet, dass** das 3-Iod-2-propinylbutylcarbamat in einem Mengenanteil von 0,01 bis 0,5 Gew.-% der Zusammensetzung vorliegt.

4. Verwendung nach einem der vorhergehenden Ansprüche, **dadurch gekennzeichnet, dass** das 3-Iod-2-propinylbutylcarbamat im Gemisch mit einem oder mehreren Lösemitteln und/oder Excipientien vorliegt.

5. Verwendung nach dem vorhergehenden Anspruch, **dadurch gekennzeichnet, dass** das Lösemittel unter den Estern einer Fettsäure und Polyethylenglykol und den Polyethylenglykolen mit einem Molekulargewicht von kleiner oder gleich 450 ausgewählt ist.

6. Verwendung nach einem der vorhergehenden Ansprüche, **dadurch gekennzeichnet, dass** die Zusammensetzung mindestens einen Wirkstoff enthält, der unter entzündungshemmenden Wirkstoffen, Antibiotika, antiseptischen Wirkstoffen und Keratolytika ausgewählt ist.

7. Verwendung nach dem vorhergehenden Anspruch, **dadurch gekennzeichnet, dass** der Wirkstoff unter α-Hydroxysäuren und β-Hydroxysäuren ausgewählt ist.

8. Verwendung nach Anspruch 6, **dadurch gekennzeichnet, dass** der antiseptische Wirkstoff Octopirox ist.

9. Verfahren zur kosmetischen Behandlung von Schuppen, das darin besteht, eine Zusammensetzung, die 3-Iod-2-propinylbutylcarbamat enthält, auf das Haar und/oder die Kopfhaut aufzubringen.

10. Kosmetische und/oder dermatologische Zusammensetzung gegen Seborrhoe, **dadurch gekennzeichnet, dass** sie in einem kosmetisch und/oder dermatologisch akzeptablen Medium eine wirksame Menge von 3-Iod-2-propinylbutylcarbamat und Octopirox enthält.

11. Zusammensetzung nach Anspruch 10, **dadurch gekennzeichnet, dass** das 3-Iod-2-propinylbutylcarbamat in einem Mengenanteil von 0,005 bis 5 % des Gesamtgewichts der Zusammensetzung vorliegt.

## Claims

1. Use of 3-iodo-2-propynyl butylcarbamate for the manufacture of a medicinal product for treating seborrhoeic dermatitis.

2. Use according to Claim 1, **characterized in that** the 3-iodo-2-propynyl butylcarbamate is present in an amount ranging from 0.005% to 5% by weight of the composition.

3. Use according to Claim 1 or 2, **characterized in that** the 3-iodo-2-propynyl butylcarbamate is present in an amount ranging from 0.01% to 0.5% by weight of the composition.

4. Use according to any one of the preceding claims, **characterized in that** the 3-iodo-2-propynyl butylcarbamate is in a mixture with one or more solvents and/or excipients.

5. Use according to the preceding claim, **characterized in that** the solvent is chosen from fatty acid esters of polyethylene glycol and polyethylene glycols with a molecular weight of less than or equal to 450.

6. Use according to any one of the preceding claims, **characterized in that** the composition comprises at least one active agent chosen from anti-inflammatory agents, antibiotics, antiseptic agents and keratolytic agents.

7. Use according to the preceding claim, **characterized in that** the active agent is chosen from α-hydroxy acids and β-hydroxy acids.

8. Use according to Claim 6, **characterized in that** the antiseptic agent is Octopirox.

9. Cosmetic process for treating dandruff, which consists in applying to the hair and/or the scalp a composition containing 3-iodo-2-propynyl butylcarbamate.

10. Anti-seborrhoeic cosmetic and/or dermatological composition, **characterized in that** it contains, in a cosmetically and/or dermatologically acceptable medium, an effective amount of 3-iodo-2-propynyl butylcarbamate and Octopirox.

11. Composition according to Claim 10, **characterized in that** the 3-iodo-2-propynyl butylcarbamate is present in an amount ranging from 0.005% to 5% relative to the total weight of the composition.
